**Europäisches Patentamt**

(19) **European Patent Office**

**Office européen des brevets**

(11) Numéro de publication: **0 052 690**

**A1**

# (12) DEMANDE DE BREVET EUROPEEN

(21) Numéro de dépôt: **80810359.2**

(22) Date de dépôt: **21.11.80**

(51) Int. Cl.³: **A 61 N 1/36**

(43) Date de publication de la demande:
**02.06.82 Bulletin 82/22**

(84) Etats contractants désignés:
**AT BE CH DE FR GB IT LI LU NL SE**

(71) Demandeur: **Precimed S.A.**
**Chemin des Tourelles 17**
**CH-2400 Le Locle(CH)**

(72) Inventeur: **Meuterlos, Louis Henri Joseph**
**Billodes 25**
**CH-2400 Le Locle(CH)**

(72) Inventeur: **Gremaud, Henri Auguste**
**Communal 8**
**CH-2400 Le Locle(CH)**

(74) Mandataire: **Tordion, Serge**
**Cabinet de Conseil en brevets 23, rue du Marché-Neuf**
**Case postale 182**
**CH-2500 Bienne 3(CH)**

(54) **Dispositif de fixation de l'électrode-sonde à la borne de sortie d'un stimulateur cardiaque.**

(57) Le dispositif de fixation de l'électrode-sonde (5) à la borne de sortie (1) du stimulateur cardiaque (2) assure l'isolation de la borne (1) et de la vis de blocage (9) de l'électrode (5) à l'aide d'un capuchon (13) vissé en usine sur le borne (1) et qui tient lieu de contre-écrou à la vis (9) jusqu'à implantation du stimulateur, pour éviter que cette vis ne sorte du taraudage (11) ou n'obstrue la forure (4) à la suite des chocs subis pendant les transports et déplacements depuis l'usine jusqu'en salle d'opération. Lors de l'implantation du stimulateur, la vis (9) est serrée sur l'électrode (5) à l'aide du tournevis (16), qui est guidé par l'ouverture (15) du capuchon (13), operculée par un coussin de silicone visqueuse (17) pris entre deux membranes souples (18, 19) incisées diamétralement (en 20), les lèvres des incisions (20) revenant dans leurs positions originales après retrait du tournevis (16).

- 1 -

## Dispositif de fixation de l'électrode-sonde à la borne de sortie d'un stimulateur cardiaque

Les stimulateurs cardiaques équipés des dispositifs de fixation connus, correspondant au préambule de la revendication 1, sont livrés aux hôpitaux, en règle générale prêts à l'implantation, dans des emballages contenant, d'une part, le stimulateur, dont la vis de blocage de l'extrémité de l'électrode-sonde est découverte, et, d'autre part, un ou plusieurs capuchons isolants ainsi que les outils permettant de serrer cette vis et de l'isoler, elle et la borne de sortie du stimulateur, en montant l'un des dits capuchons sur l'appareil.

Dans ces cas, c'est donc au chirurgien qu'il incombe de monter ce capuchon sur l'appareil, après qu'il en ait serré la vis de blocage sur l'extrémité de l'électrode-sonde, préalablement mise en place dans le corps du patient.

Sous cette forme, le dispositif de fixation de l'électrode-sonde au stimulateur présente plusieurs inconvénients. Il n'est tout d'abord pas exclu que la vis de blocage de l'électrode-sonde, qui est entièrement libre dans la borne de sortie du stimulateur, ne s'en échappe par suite des chocs que l'emballage de l'appareil subit au cours des transports et déplacements auxquels il est soumis depuis l'instant où il est scellé chez le fabricant jusqu'au moment où il est ouvert en salle d'opération. Or, dans une telle éventualité, la vis en question risquerait fort de se perdre lors de l'ouverture de l'emballage de l'appareil. Il pourrait même arriver que le chirurgien termine l'implantation du stimulateur sans s'apercevoir de la disparition de cette vis, ce qui compromettrait fort les effets du stimulateur, en raison du contact très aléatoire entre l'électrode-sonde et la borne de sortie du stimulateur. Et si, par ailleurs, la vis de blocage de l'électrode-sonde devait être retrouvée dans l'emballage de l'appareil, lors de son ouverture, et être dûment identifiée, le personnel du bloc opératoire serait bien emprunté pour la remettre en place, avec les mains gantées et sans les outils adéquats.

Même sans accident de ce genre, la mise en place du capuchon isolant la vis de blocage de l'électrode-sonde et la borne de sortie du stimulateur est une opération délicate pour le chirurgien ganté. Même si un assistant lui passe le tournevis enfiché dans une ouverture polygonale ou étoilée de ce capuchon, le risque demeure ou bien que le capuchon tombe du tournevis et se perde ou alors qu'il soit engagé de travers dans son taraudage ou encore que ses filets soient arrachés, si le tournevis n'est pas muni d'un dispositif limiteur de couple entre le manche et la lame ou si ce dispositif limiteur ne fonctionne pas bien ou si le tournevis est manipulé maladroitement. Dans tous ces cas, il en résulterait que le capuchon ne garantirait pas l'isolation de la borne de sortie du stimulateur, de sorte que les impulsions de ce dernier ne parviendraient pas ou qu'imparfaitement au cœur.

Il existe sur le marché des stimulateurs qui dispensent le chirurgien de cette dernière opération par le fait que le capuchon recouvrant la borne de sortie du stimulateur et sa vis de blocage de l'électrode-sonde est fixé à demeure à l'appareil lors de sa fabrication. Juste avant l'implantation de ce dernier, un assistant doit alors percer un trou au centre du capuchon. Le chirurgien n'a plus alors qu'à serrer la vis de blocage de l'électrode-sonde en passant un tournevis à travers ce trou.

Dans ce cas, l'isolation de la borne de sortie du stimulateur n'est cependant plus garantie et la proportion de l'intensité de l'impulsion émise par le stimulateur qui risque d'être court-circuitée par le sang et les sécrétions s'infiltrant à travers le trou pratiqué dans le capuchon est tout à fait indéterminée, ce qui compromet évidemment les effets du stimulateur sur le patient. Ce risque est encore accru, si le trou du capuchon n'est pas percé au bon endroit et qu'il faille l'agrandir pour pouvoir actionner la vis de blocage de l'électrode-sonde. Par ailleurs, le capuchon de ces dispositifs connus n'empêche pas la vis de blocage de l'électrode-sonde de sortir accidentellement de son taraudage et de se placer en travers de la borne de sortie du stimulateur, dans la cavité comprise entre cette borne et le capuchon.

Le but de l'invention est de créer un disposi-

- 4 -

tif de fixation de l'électrode-sonde à la borne de sortie d'un stimulateur cardiaque qui soit à l'abri des inconvénients mentionnés ci-dessus des dispositifs connus, tout en permettant au chirurgien d'effectuer l'implantation du stimulateur sans retarder son travail par des manipulations relevant d'une technique avec laquelle il n'est pas familiarisé.

Grâce aux particularités définies par la caractéristique de la revendication 1, ce but est atteint : le risque de perdre la vis de blocage de l'électrode-sonde est éliminé; le chirurgien n'a plus à s'embarrasser de la mise en place du capuchon isolant; le serrage de la vis de blocage de l'électrode-sonde est la seule opération mécanique dont le chirurgien ait encore à s'occuper. Les opérations extramédicales pour l'implantation du stimulateur sont ainsi réduites au minimum indispensable. Le dispositif défini par la revendication 1 a, de surcroît, l'avantage que l'isolation de la borne de sortie du stimulateur est réalisée par le fabricant de l'appareil, c'est-à-dire par des personnes manifestement mieux outillées et plus compétentes pour ce travail que le personnel d'un bloc opératoire.

Les formes spéciales d'exécution définies par la revendication 2 ont l'avantage d'éviter que la vis de blocage de l'électrode-sonde n'obstrue la forure de la borne de sortie du stimulateur et n'empêche l'introduction convenable de l'extrémité de l'électrode-sonde lors de l'implantation du stimulateur, au risque que le chirurgien ne serre cette vis dans le vide, devant l'extrémité de l'électro-

0052690

- 5 -

de-sonde et non sur celle-ci.

La bague métallique définie par la revendication 3 a l'avantage de retenir en place fermement l'opercule obturant l'ouverture centrale du capuchon et en même temps de maintenir la vis de blocage de l'électrode-sonde éloignée de la forure destinée à recevoir l'extrémité de cette électrode jusqu'au moment de l'implantation du stimulateur.

Quant à la revendication 4, elle définit un opercule particulièrement avantageux pour garantir l'isolation de la borne de sortie du stimulateur, une fois que celui-ci est implanté.

Enfin, l'ouverture centrale du capuchon, qui est définie par la revendication 5, que le fabricant de l'appareil n'a nulle peine à centrer sur l'axe de la vis de blocage engagée dans la borne de sortie du stimulateur, du fait que le capuchon est vissé sur cette borne, a l'avantage de guider sûrement le tournevis dans l'entrée de cette vis.

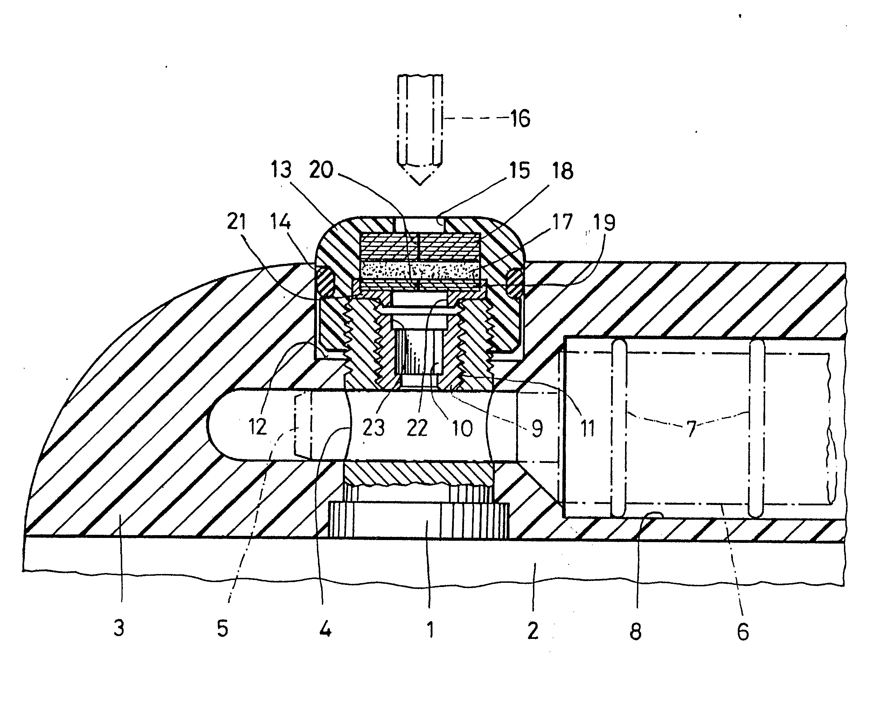

Une forme d'exécution du dispositif de fixation selon l'invention est représentée schématiquement et à titre d'exemple dans la figure unique du dessin, qui en est une coupe.

La borne de sortie 1 du stimulateur 2 est enrobée partiellement, de façon usuelle, dans un connecteur 3 qui surmonte le stimulateur. La borne 1 présente une forure transversale 4, destinée à recevoir l'extrémité d'une électrode-sonde 5 conventionnelle. Comme à l'accoutumée, cette dernière est entourée d'un manchon 6 en matière isolante, muni de bourrelets 7, qui pénètre dans un alésage 8 du connecteur 3. Cet alésage guide l'extrémité de

- 6 -

l'électrode-sonde 5 dans la forure 4. Quant aux bourrelets 7, ils empêchent le sang et toute autre sécrétion du corps de s'infiltrer dans l'alésage 8 jusqu'à la partie dénudée de l'électrode-sonde 5. Ils assurent par conséquent l'isolation de la connexion de l'électrode-sonde 5 à la borne 1 du côté de l'alésage 8.

Une vis de blocage 9, présentant une ouverture de section polygonale 10 et engagée dans un taraudage 11 de la borne 1, retient l'électrode-sonde 5 dans la forure 4. Pour assurer l'accès à la vis 9, l'extrémité de la borne 1 débouche dans une creusure 12 du connecteur 3.

L'isolation de cette extrémité de la borne 1 et de la vis 9 est assurée par un capuchon 13, qui est vissé sur la borne 1. Une garniture 14 empêche toute infiltration entre le capuchon 13 et la paroi de la creusure 12. Le capuchon 13 présente une ouverture centrale circulaire 15, qui est donc coaxiale à la vis 9. Le diamètre de l'ouverture 15 est approximativement égal à celui du cercle circonscrit à l'entrée de la section polygonale 10 de l'ouverture de la vis 9, afin de guider l'extrémité prismatique d'un tournevis 16 dans l'ouverture 10 de la vis 9. L'ouverture 15 du capuchon 13 est operculée par un coussin 17 de silicone visqueuse, qui est emprisonné entre deux membranes souples 18 et 19 en silicone caoutchouteuse. Chacune de ces membranes présente une incision diamétrale 20 dans sa partie centrale, qui est assez longue pour livrer passage sans risque de déchirure au tournevis 16. Les membranes 18 et 19 sont retenues dans le capuchon 13 par une bague métallique 21. Lors du montage de ces membranes, il n'est pas nécessaire que leurs incisions 20 soient orientées de la même façon; il est même préférable que ces incisions soient croisées. La bague 21 présente une ouverture 22 de diamètre inférieur au taraudage 11 de la borne 1. Cette bague 21 fait ainsi saillie à l'intérieur du taraudage 11.

Le capuchon operculé de la façon décrite garantit l'isolation de la vis 9 et de la borne 1. Après avoir traversé cinquante fois l'opercule 17, 18, 19 à l'aide du tournevis 16, cet opercule résistait encore à des pressions montant jusqu'à quatre atmosphères. Or, en pratique, cet opercule n'est destiné à être traversé que trois ou quatre fois au plus.

Le capuchon 13, équipé de la façon décrite, est vissé une fois pour toutes sur la borne 1, lors de l'assemblage de l'appareil, c'est-à-dire par le fabricant de ce dernier. La bague 21 est alors appuyée contre l'extrémité de la borne 11. Quant à la vis 9, qui est ainsi emprisonnée dans le taraudage 11, elle est dévissée jusqu'à ce qu'elle soit bloquée contre la bague 21, qui fait office de contre-écrou. Dans cet état, le stimulateur peut être transporté sans danger de la fabrique aux hôpitaux. La vis 9 ne risque pas de se déplacer et d'obstruer la forure 4.

Lors de l'implantation du stimulateur, le chirurgien, après avoir mis en place l'électrode-sonde dans le corps du patient, n'a plus qu'à en introduire le manchon terminal 6 dans l'alésage 8 du connecteur 3 jusqu'à ce qu'il voie l'extrémité de l'électrode 5 sortir de la borne 1 à travers les parois du connecteur 3, qui est fait en matière transparente. A l'aide du tournevis, qui se trouve dans l'emballage du stimulateur, il lui suffit alors de serrer la vis 9 sur l'électrode 5 en le faisant passer par l'ouverture 15 à travers l'opercule 17, 18, 19.

Le dessin montre que la partie polygonale 10 de l'ouverture de la vis 9 ne s'étend pas jusqu'à l'entrée de cette ouverture. Dans sa partie supérieure, la vis 9 présente, en effet, un dégagement 23, afin que les lèvres de l'incision 20 de la membrane 19 ne risquent pas de se coincer entre le tournevis 16 et les pans de l'ouverture 10 de la vis 9 et d'être déchirées.

REVENDICATIONS:

1. Dispositif de fixation de l'électrode-sonde à la borne de sortie d'un stimulateur cardiaque, dans lequel l'extrémité de l'électrode-sonde est guidée vers une forure transversale de la dite borne par un alésage d'un connecteur en matière isolante qui surmonte le stimulateur, enrobe partiellement sa borne de sortie et présente une creusure au fond de laquelle débouche cette borne pour donner accès à une vis de blocage de l'extrémité de l'électrode-sonde dans la dite forure, cette vis de blocage et la dite borne étant isolées électriquement du milieu ambiant par un capuchon les recouvrant,
caractérisé
- en ce que ce capuchon (13) est vissé définitivement sur la borne de sortie (1) du stimulateur (2) lors de l'assemblage de ce dernier,
- en ce qu'il empêche la vis de blocage (9) de l'électrode-sonde (5) de sortir de son taraudage (11)
- et en ce qu'il présente une ouverture centrale (15) obturée par un opercule (17, 18, 19) qui laisse passer l'extrémité du tournevis (16) destiné à actionner la vis de blocage de l'électrode-sonde et se referme après le retrait du tournevis.

2. Dispositif de fixation selon la revendication 1,
caractérisé
en ce que le dit capuchon (13) tient lieu de contre-écrou à la vis de blocage (9) de l'électrode-sonde (5) avant l'implantation du stimulateur (2).

3.    Dispositif de fixation selon la revendication 2,

caractérisé

en ce que le dit capuchon (13) porte une bague métallique (21) qui retient le dit opercule (17, 18, 19) et fait saillie à l'intérieur du taraudage (11) de la borne de sortie (1) du stimulateur (2) prévu pour la dite vis de blocage (9), cette dernière étant dévissée jusqu'à appui contre la dite bague lors de l'assemblage du stimulateur.

4.    Dispositif de fixation selon la revendication 1 ou la revendication 2 ou la revendication 3, caractérisé

en ce que l'opercule est constitué par un coussin de silicone visqueuse (17) pris entre deux membranes souples (18, 19), incisées (20) diamétralement.

5.    Dispositif de fixation selon l'une ou l'autre des revendications précédentes, caractérisé

en ce que l'ouverture centrale (15) du capuchon (13) a un diamètre approximativement égal à celui du cercle circonscrit à l'entrée polygonale (10) de la vis de blocage (9) de l'électrode-sonde (5).

**0052690**

---

Office européen
des brevets

**RAPPORT DE RECHERCHE EUROPEENNE**

**0052690**
Numéro de la demande

EP 80 81 0359

| DOCUMENTS CONSIDERES COMME PERTINENTS | | | CLASSEMENT DE LA DEMANDE (Int. Cl.³) |
|---|---|---|---|
| Catégorie | Citation du document avec indication, en cas de besoin, des parties pertinentes | Revendication concernée | |
| | FR - A - 2 268 510 (MEDTRONIC) <br><br> * page 10, lignes 4-26; page 11, lignes 12-20; page 13, lignes 16-18 * <br><br> -- | 1,2 | A 61 N 1/36 <br> H 01 R 4/36 |
| | US - A - 4 010 760 (MEDTRONIC) <br><br> * colonne 3, lignes 24-36; figures 1,3 * <br><br> -- | 1,2 | |
| | US - A - 3 198 195 (CHARDACK) <br><br> * colonne 3, lignes 45-61; figure 2 * <br><br> -- | 1,4 | **DOMAINES TECHNIQUES RECHERCHES (Int. Cl.³)** <br><br> A 61 N  1/36 <br> H 01 R  4/36 <br> F 16 B 41/00 |
| | US - A - 4 015 890 (HECO) <br><br> * colonne 2, lignes 21-29; colonne 2, ligne 61 - colonne 3, ligne 6 * <br><br> -- | 2,5 | |
| | FR - A - 1 251 307 (LEGRAND) <br><br> * page 1, colonne de droite, alinéas 2-4 * <br><br> -- | 2,5 | **CATEGORIE DES DOCUMENTS CITES** |
| | FR - A - 2 219 535 (WIELAND) <br><br> * page 3, lignes 1-11 * <br><br> -- | 4 | X: particulièrement pertinent <br> A: arrière-plan technologique <br> O: divulgation non-écrite <br> P: document intercalaire <br> T: théorie ou principe à la base de l'invention |
| A | US - A - 4 141 752 (SHIPKO) <br><br> ------ | | E: demande faisant interférence <br> D: document cité dans la demande <br> L: document cité pour d'autres raisons |
| | Le présent rapport de recherche a été établi pour toutes les revendications | | &: membre de la même famille, document correspondant |
| Lieu de la recherche <br> La Haye | Date d'achèvement de la recherche <br> 25-08-1981 | Examinateur <br> SIMON | |

OEB Form 1503.1  06.78